(19) Europäisches Patentamt
European Patent Office
Office européen des brevets

(11) Numéro de publication: **0 297 947 B1**

(12) **FASCICULE DE BREVET EUROPEEN**

(45) Date de publication du fascicule du brevet: **16.01.91**

(51) Int. Cl.⁵: **C 07 C 19/08,** C 07 C 17/20

(21) Numéro de dépôt: **88401533.0**

(22) Date de dépôt: **20.06.88**

(54) **Synthèse du chloro-1-difluoro-1,1-éthane.**

(30) Priorité: **03.07.87 FR 8709495**

(43) Date de publication de la demande:
**04.01.89 Bulletin 89/01**

(45) Mention de la délivrance du brevet:
**16.01.91 Bulletin 91/03**

(84) Etats contractants désignés:
**AT BE DE ES FR GB GR IT NL SE**

(56) Documents cités:
**DE-A-2 659 046**

(73) Titulaire: **ATOCHEM**
**4 & 8, Cours Michelet La Défense 10**
**F-92800 Puteaux (FR)**

(72) Inventeur: **Cheminal, Bernard**
**Lieu-dit La Rivière Orlienas**
**F-69530 Brignais (FR)**
Inventeur: **Lantz, André**
**Domaine de la Hêtraie**
**F-69390 Vernaison (FR)**

(74) Mandataire: **Leboulenger, Jean et al**
**ATOCHEM Département Propriété Industrielle**
**F-92091 Paris la Défense 10 Cédex 42 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 297 947 B1

Courier Press, Leamington Spa, England.

**Description**

La présente invention concerne la fabrication du chlor-1 difluoro-1,1 éthane $CF_2Cl—CH_3$ par réaction catalytique en phase liquide du trichloro-1,1,1 éthane $CCl_3—CH_3$ avec l'acide fluorhydrique HF.

Le chloro-1 difluoro-1,1 éthane qui est surtout utilsé comme intermédiaire dans la synthèse du difluoro-1,1 éthylène $CF_2=CH_2$ Lui-même monomère d'importance croissante pour l'élaboration industrielle de polymères fluorés), mais aussi comme propulseur d'aérosol, est en général préparé par réaction de trichloro-1,1,1 éthane et d'acide fluorhydrique en phase liquide.

Selon un procédé connu (brevet DE 2 137 806), la réaction peut être effectuée sand catalyseur à condition d'opérer à température élevée (110°C) avec un grand excès d'acide fluorhydrique (rapport molaire $HF/C_2H_3Cl_3$ compris entre 15 et 30). Ceci conduit à une sous-production importante de trifluoro-1,1,1 éthane et à une faible productivé en chloro-1 difluoro-1,1 éthane. De plus, l'entraînement d'acide fluorhydrique par l'acide chlorhydrique formé dand cette réaction d'échange chlor <—> fluor nécessite un appareillage adapté (sous pression suffisante) pour la récupération de cette matière première coûteuse. Dans le brevet précité, il est indiqué que l'emploi d'acide $HSO_3F$ conduit à la formation de goudrons, probablement du à l'instabilité chimique de ce catalyseur (oxydation par $SO_3$).

D'autres procédés connus préconisent l'utilisation de catalyseurs à base de dérivés de l'antimoine ou du molybdène (voir par exemple, le brevet DE 2 659 046 et les publications japonaises 74—03965, 76—29404 et 76—39606).

L'inconvénient majeur des dérivés d'antimoine réside dans leur désactivation rapide par réduction en valence inférieure au degré d'oxydation $Sb^{3+}$. La parade qui consiste à oxyder en continu le catalyseur (à l'aide de chlore) contribue à la formation de composés lourds (dérivés d'une chloration du groupe $CH_3$ du trichloro-1,1,1 éthane). De plus, la solubilité de certaines espèces catalytiques ($SbF_3$ par exemple) est tellement faible dans le milieu réactionnel qu'elle entraîne le plus souvent une séparation physique de cette espèce et l'inefficacité de l'action du chlore. Ceci se traduit par une irrégularité de la réaction chimique et une consommation importante de catalyseur, ainsi qu'une corrosion soutenue due à la formation de composés superacides trés agressifs vis-à-vis du matériau constituant le réacteur. Enfin, la difficulté à régler convenablement l'activité catalytique conduit à une importante sous-production de trifluoro-1,1,1 éthane indésirable.

Acec les dérivés du molybdène, la formation d'espèces volatiles entraîne une perte de catalyseur dans la phase gazeuse du réacteur.

Il a maintenant été trouvé qu'on peut remédier à ces inconvénients et réaliser la réaction avec un excès raisonnable d'acide fluorhydrique (rapport molaire $HF/CH_3—CCl_3$ compris entre 2 et 5), en utilisant un catalyseur acide soluble dans le milieu réactionnel et constitué par un acide perfluoroalcane-sulfonique $CF_3—(CF_2)_n—SO_3H$, n pouvant aller de 0 à 7 et étant de préférence égal à zéro (acide triflique $CF_3—SO_3H$).

Le catalyseur selon l'invention est avantageusement utilisé à raison de 1 à 10 moles pour 100 moles de trichloro-1,1,1 éthane et, de préférence, à raison de 3 à 5 moles pour 100.

Le procédé selon l'invention peut être mis en oeuvre en discontinu ou en continu, à une température allant de 80 à 150°C, mais de préférence voisine de 100—110°C.

En discontinu, on opère dans un réacteur fermé dans lequel tous les réactifs sont introduits au début de l'opération et où la pression autogène augment jusqu'à une valeur maximale.

En continu, les réactifs sont introduits au moyen d'une pompe dans le mélange réactionnel contenant le catalyseur avec récupération continue des composés utiles (chloro-1 difluoro-1,1 éthane et acide chlorhydrique) et condensation des composés plus lourds (catalyseur, acide fluorhydrique, trichloro-1,1,1 éthane, dichloro-1,1 fluoro-1 éthane), tout en maintenant la pression totale à une valeur déterminée au moyen d'un dispositif régulateur approprié. La pression de travail doit être suffisante (au moins égale à 15 bars et, de préférence, comprise entre 15 et 20 bars) pour permettre d'une part la séparation de l'acide chlorhydrique par distillation et d'autre part pour maintenir les réactifs à l'état liquide.

Le dichloro-1,1 fluoro-1 éthane formé est avantageusement recyclé pour être transformé en chloro-1 difluro-1,1, éthane.

Les exemples suivants, dans lesquels les pourcentages indiqués sont exprimés en moles, illustrent l'invention.

Exemple 1

Dans un autoclave en acier inoxydable (NS 22 S) d'un volume de 800 ml, muni d'un système d'agitation par barreau magnétique, on introduit successivement 3,2 g d'acide triflique, 71 g de trichloro-1,1,1 éthane et 23,3 g d'acide fluohydrique.

L'agitation fonctionnant à environ 700 tr/min, on porte la mélange réactionnel à 100°C par l'intermédiaire d'un fluide caloporteur circulant dans la double enveloppe du réacteur. La pression maximale autogène atteint 31,2 bars (soit environ 32 bars absolus).

Après une heure de réaction dans ces conditions, le mélange est refroidi sous agitation, ce qui requiert environ 50 minutes pour atteindre la température ambiante.

L'analyse des hydracides et des composés organiques du mélange réactionnel donne les résultats suivants:

- taux de conversion du trichloro-1,1,1 éthane = 92,6 %
- taux de transformation du $CH_3$-$CCl_3$
  - en $CH_3$-$CF_3$ = 0,07 %
  - en $CH_3$-$CF_2Cl$ = 12,3 %
  - en $CH_3$-$CFCl_2$ = 79,2 %
- ratio $CF_3$-$CH_3$/$CF_2Cl$-$CH_3$ = 0,6 %

Exemple 2

On répète l'exemple 1, mais sans agitation. La pression autogène maximale attient 31 bars absolus et on obtient les résultants suivants:

- taux de conversion du $CH_3$-$CCl_3$ = 89 %
- taux de transformation du $CH_3$-$CCl_3$
  - en $CH_3$-$CF_3$ = 0,13 %
  - en $CH_3$-$CF_2Cl$ = 14,5 %
  - en $CH_3$-$CFCl_2$ = 71,7 %
- ratio $CF_3$-$CH_3$/$CF_2Cl$-$CH_3$ = 0,9 %/

Ce dernier ratio monte qu'il est préférable de réaliser la réaction sous agitation.

Exemple 3

On répète l'exemple 1, mais on modifie le rapport molaire HF/$CH_3$—$CCl_3$ pour l'amener 2,2 à 5 en utilisant les quantités suivantes de réactifs:

— 2,8 g d'acide triflique
— 61,8 g de trichloro-1,1,1 éthane
— 46,3 g d'acide fluorohydrique

Dans cet essai réalisé dans des conditions identiques à celles de l'exemple 1 (agitation à 700 tr/min; 100°C), la pression atuogène maximale atteint 36 bars absolus et les résultats obtenus sount les suivants:

- taux de conversion du $CH_3$-$CCl_3$ = 93,5 %
- taux de transformation du $CH_3$-$CCl_3$
  - en $CH_3$-$CF_3$ = 0,4 %
  - en $CH_3$-$CF_2Cl$ = 37,2 %
  - en $CH_3$-$CFCl_2$ = 52,6 %
- ratio $CH_3$-$CF_3$/$CH_3$-$CF_2Cl$ = 1,1 %

Essai comparatif n° 1

On opère comme à l'exemple 1, mais sans addition de catalyseur; les quantités de réactifs engagés sont les suivantes:

— 66,8 g de trichloro-1,1,1 éthane
— 21,9 g d'acide fluorhydrique

La pression autogène maximale atteint 27 bars absolus et on obtient les résultats suivants:

- taux de conversion du $CH_3$-$CCl_3$ = 81,2 %
- taux de transformation du $CH_3$-$CCl_3$
  - en $CH_3$-$CF_3$ = 0,03 %
  - en $CH_3$-$CF_2Cl$ = 2,9 %
  - en $CH_3$-$CFCl_2$ = 78,0 %
- ratio $CH_3$-$CF_3$/$CH_3$-$CF_2Cl$ = 1 %

La comparaison des résultats de cet essai avec ceux obtenus à l'exemple 1 montre que le catalyseur selon l'invention permet d'améliorer la sélectivité en $CH_3$—$CF_2Cl$ alors même que le taux de transformation de $CH_3$—$CCl_3$ en $CH_3$—$CF_2Cl$ est quatre fois plus important.

Essai comparatif n° 2

Cet essai a été réalisé en présence de $SbCl_5$ comme catalyseur et à une température de 65°C avec les réactifs suivants, introduits dans l'ordre indiqué:

- 6 g de $SbCl_5$
- 66,8 g de $CH_3$-$CCl_3$
- 21,9 g de HF

Les résultats obtenus en une heure et sous agitation à 700 tr/min (pression maximale atteinte: 36 bars absolus) sont les suivants:

- taux de conversion du $CH_3$-$CCl_3$ = 98,5 %
- taux de transformation du $CH_3$-$CCl_3$
  - en $CH_3$-$CF_3$ = 28,4 %
  - en $CH_3$-$CF_2Cl$ = 64,4 %
  - en $CH_3$-$CFCl_2$ = 1,7 %
- ratio $CH_3$-$CF_3$/$CH_3$-$CF_2Cl$ = 44,1 %

Bien que réalisé à une température plus basse qui défavorise la formation de $CH_3$—$CF_3$, cet essai montre bien l'influence de la nature du catalyseur sur la sélectivé recherchée en $CH_3$—$CF_2Cl$.

## Revendications

1. Procédé de fabrication du chlor-1 difluoro-1,1 éthane en phase liquide par action de l'acide fluorhydrique sur le trichloro-1,1,1 éthane, caractérisé en ce qu'on utilise comme catalyseur un acide perfluoroalcane-sulfonique $CF_3$—$(CF_2)_n$—$SO_3H$, n étant un nombre entier allant de 0 à 7.

2. Procédé selon la revendication 1, dans lequel le catalyseur est l'acide triflique.

3. Procédé selon la revendication 1 ou 2, dans lequel on utilise 1 à 10 moles de catalyseur pour 100 moles de trichloro-1,1,1 éthane, de préférence 3 à 5 moles pour 100.

4. Procédé selon l'une des revendications 1 à 3, dans lequel le réaction est effectuée à une température allant de 80 à 150°C, de préférence entre environ 100 et 110°C.

5. Procédé selon l'une des revendications 1 à 4, dans lequel le rapport molaire HF/$CH_3$—$CCl_3$ est compris entre 2 et 5.

6. Procédé selon l'une des revendications 1 à 5, dans lequel la pression totale est comprise entre 15 et 50 bars.

7. Procédé selon l'une des revendications 1 à 6, dans lequel on opère sous agitation.

8. Procédé selon l'une des revendications 1 à 7, dans lequel on opère de manière discontinue sous la pression autogène du mélange réactionnel.

9. Procédé selon l'une des revendications 1 à 7, dans lequel on opère de manière continue sous une pression comprise entre 15 et 20 bars.

10. Procédé selon l'une des revendications 1 à 9, dans lequel le dichloro-1,1 fluoro-1 éthane formé est recyclé.

## Patentansprüche

1. Verfahren zur Herstellung von 1-Chlor-1,1-difluorethan durch Einwirken von Fluorwasserstoffsäure auf 1,1,1-Trichlorethan in der Flüssigphase, dadurch gekennzeichnet, daß man als Katalysator eine Perfluoroalkansulfonsäure, $CF_3$—$(CF_2)_n$—$SO_3H$, worin n eine ganze Zahl von 0 bis 7 ist, einsetzt.

2. Verfahren nach Anspruch 1, in dem der Katalysator Trifluoromethansulfonsäure ist.

3. Verfahren nach Anspruch 1 oder 2, in dem man 1 bis 10 mol Katalysator pro 100 mol und vorzugsweise 3 bis 5 mol pro 100 mol 1,1,1-Trichloroethan einsetzt.

4. Verfahren nach einem der Ansprüche 1 bis 3, in dem die Umsetzung bei einer Temperatur von 80°C bis 150°C und vorzugsweise zwischen etwa 100°C und 110°C durchgeführt wird.

5. Verfahren nach einem der Ansprüche 1 bis 4, in dem das Molverhältnis HF/$CH_3$—$CCl_3$ zwischen 2 und 5 beträgt.

6. Verfahren nach einem der Ansprüche 1 bis 5, in dem der Gesamtdruck zwischen 15 und 50 bar beträgt.

7. Verfahren nach einem der Ansprüche 1 bis 6, in dem man unter Rühren arbeitet.

8. Verfahren nach einem der Ansprüche 1 bis 7, in dem man diskontinuierlich bei dem autogenen Druck des Reaktionsgemisches arbeitet.

9. Verfahren nach einem der Ansprüche 1 bis 7, in dem man kontinuierlich bei einem Druck zwischen 15 und 20 bar arbeitet.

10. Verfahren nach einem der Ansprüche 1 bis 9, in dem das gebildete 1,1-Dichlor-1-fluorethan in den Kreislauf zurückgeführt wird.

## Claims

1. Process for manufacturing 1-chloro-1,1-difluoroethane in the liquid phase by the action of hydrofluoric acid on 1,1,1-trichloroethane, characterized in that a perfluoroalkanesulphonic acid $CF_3-(CF_2)_n-SO_3H$, n being an integer ranging from 0 to 7, is used as a catalyst.

2. Process according to Claim 1, in which the catalyst is triflic acid.

3. Process according to Claim 1 or 2, in which 1 to 10 moles of catalyst per 100 moles of 1,1,1-trichloroethane, and preferably 3 to 5 moles per 100, are used.

4. Process according to Claims 1 to 3, in which the reaction is performed at a temperature ranging from 80 to 150°C, and preferably between approximately 100 and 110°C.

5. Process according to one of Claims 1 to 4, in which the $HF/CH_3-CCl_3$ mole ratio is between 2 and 5.

6. Process according to one of Claims 1 to 5, in which the total pressure is between 15 and 50 bars.

7. Process according to one of Claims 1 to 6, in which the reaction is performed with stirring.

8. Process according to one of Claims 1 to 7, in which the reaction is performed in discontinuous fashion under the autogenous pressure of the reaction mixture.

9. Process according to one of Claims 1 to 7, in which the reaction is performed in continuous fashion under a pressure of between 15 and 20 bars.

10. Process according to one of Claims 1 to 9, in which the 1,1-dichloro-1-fluoroethane formed is recycled.